# EUROPÄISCHE PATENTSCHRIFT

(11) **EP 0 750 587 B1**
(45) Veröffentlichungstag und Bekanntmachung des Hinweises auf die Patenterteilung: **12.01.2000**
(21) Anmeldenummer: 95912221.9
(22) Anmeldetag: 08.03.1995
(51) Int. Cl.: C01B 25/26, B01J 29/82

(54) **KRISTALLINE BOROPHOSPHATE, IHRE HERSTELLUNG UND VERWENDUNG**
CRYSTALLINE BOROPHOSPHATES, THEIR PRODUCTION AND USE
BOROPHOSPHATES CRISTALLINS, LEUR PRODUCTION ET LEUR UTILISATION

(30) Priorität: 14.03.1994 DE 4408486
(43) Veröffentlichungstag der Anmeldung: 02.01.1997
(73) Patentinhaber: BASF AKTIENGESELLSCHAFT, 67056 Ludwigshafen (DE)
(72) Erfinder: KNIEP, Rüdiger, D-40764 Langenfeld (DE)
(86) Internationale Anmeldenummer: EP9500846
(87) Internationale Veröffentlichungsnummer: WO9525066

(56) Entgegenhaltungen:
- US-A- 4 298 503
- Derwent's abstract, Nr. 86-345798/52, Woche 8652; & SU,A,1234358, (BELORUSS KIROV TECHN INS), 1986-05-30

## Beschreibung

Die Erfindung betrifft kristalline Borophosphate ein- und/oder mehrwertiger Kationen, deren Herstellung und Verwendung, beispielsweise als Ionenaustauscher und Sorptionsmittel, als keramische Materialien für den medizinischen Bereich, vorzugsweise als Katalysatoren.

Man kennt eine Vielzahl kristalliner anorganischer Festkörper, die oligomere oder polymere Anionenverbände enthalten, die durch eckenverknüpfte Sauerstofftetraeder mit Metall- oder Nichtmetall Atomen im Zentrum aufgebaut sind. Hierzu gehören beispielsweise die Substanzklassen der Zeolithe und der Aluminiumphosphate, die als Katalysatoren, als Ionenaustauscher und Molekularsiebe beträchtliche technische Bedeutung haben. Aus der sehr umfangreichen Literatur hierzu sei beispielsweise zitiert: A. Tißler, U. Müller und K. K. Unger, "Synthetische Zeolithe und Aluminophosphate", Nachr. Chem. Techn. Lab. 36 (6), S.624-630 (1988).

Für viele technische Anwendungen dieser Festkörper ist der dreidimensional-periodische Aufbau von Bedeutung, der durch die Bezeichnung "kristallin" wiedergegeben wird. Im Sinne der vorliegenden Schrift wird "kristallin" dadurch definiert, daß die Festkörper beim Bestrahlen mit Röntgenstrahlen ein Röntgenbeugungsdiagramm liefern, das diskrete Beugungsmaxima aufweist.

Kristalline Borophosphate, die oligomere oder polymere Bor- und Phosphorhaltige Anionenverbände enthalten, sind im Stand der Technik kaum, ihre technische Verwendung überhaupt nicht beschrieben. Die Herstellung kristalliner Phasen der Zusammensetzung 2 M0.P₂0₅.B₂0₃, wobei M für die Erdalkalimetalle Calcium, Strontium und Barium steht, durch eine Hochtemperatursynthese wurde von H. Bauer veröffentlicht (Z. anorg. allg. Chem. 345, S.225-229 (1966), Z. anorg. allg. Chem. 337, S.183-190 (1965)). Alternative Herstellverfahren sowie der technische Einsatz kristalliner Borophosphate waren bisher nicht bekannt.

Bei einer thermochemischen Untersuchung des Dreistoffsystems CaO-B₂0₃-P₂0₅ konnten P. Ramamoorthy und T. J. Rockett (J. Am. Ceram. Soc. 57, S. 501-502 (1974) bei 900 °C als einzige ternäre Phase nur das vorstehend erwähnte 2Ca0.P₂0₅.B₂0₃ beobachten. Von J. Liebertz und S. Stähr (Z. Krist. 160, 135-137 (1982)) wurden die Existenz von Zn₃BPO₇ und Mg₃BPO₇ nachgewiesen und die Gitterkonstanten dieser Festkörper bestimmt.

Aus Amer. Mineral 76, S. 1400-1407 (1991) ist die Struktur des Minerals Lüneburgit bekannt, dessen Zusammensetzung in Oxidschreibweise als

3MgO. B₂0₃ P₂0₅. 9H₂0

formuliert werden kann. Der Artikel zitiert ein weiteres Mineral, Seamanit, mit der Oxidformel

6MnO. B₂0₂.P₂0₅ 6H₂0.

Die Erfindung stellt sich die Aufgabe, neue kristalline Borophosphate zur Verfügung zu stellen und alternative Herstellverfahren sowie technische Einsatzgebiete aufzuzeigen.

Die Erfindung umfaßt demnach in einer ersten Ausführungsform kristalline Borophosphate und Verfahren zur Herstellung von kristallinen Borophosphaten ein- und/oder mehrwertiger Kationen der allgemeinen Formel (I)

x M₂/ₙO · y B₂O₃ · P₂O₅ · m H₂O (I)

wobei
- n: 1, 2 oder 3 bedeutet und die Wertigkeit des Metalls M ausdrückt,
- y: zwischen 0,1 und 50 liegt,
- m: eine Zahl im Bereich von O bis (2 + y) bedeutet,
- x: so gewählt wird, daß n·x im Bereich 0,1 bis 6.(1 + y) liegt und,
- M: Natrium oder Kalium bedeutet,
dadurch gekennzeichnet, daß sie ein Röntgenpulverdiagramm mit mindestens den folgenden Linien aufweisen:

| d(Å) | I/Iₒ |
|---|---|
| 5,820 | 72 |
| 4,917 | 17 |
| 4,029 | 31 |
| 3,861 | 100 |
| 3,677 | 23 |
| 3,609 | 27 |
| 3,370 | 12 |
| 3,317 | 31 |
| 3,188 | 18 |
| 3,080 | 10 |
| 2,922 | 14 |
| 2,889 | 29 |
| 2,570 | 22 |
| 2,439 | 17 |
| 2,249 | 17 |
| 2,234 | 18 |
| 2,157 | 12 |
| 2,081 | 12 |
| 1,896 | 14 |
| oder | |

| d(Å) | I/Iₒ |
|---|---|
| 5,562 | 73 |
| 3,734 | 68 |
| 3,517 | 100 |
| 3,356 | 92 |
| 2,910 | 55 |
| 2,760 | 72 |
| 2,170 | 38 |

Als Quelle dieser Metall-Kationen M kommen solche Verbindungen in Betracht, aus denen im wäßrigen Milieu die Metall-Kationen freigesetzt werden können. Dies sind insbesondere die wasserlöslichen Salze, vorzugsweise Borate und Phosphate, aber auch Salze weiterer anorganischer oder organischer Säuren, organische Komplexverbindungen wie beispielsweise Acetylacetonate sowie Oxide und Hydroxide. Als Quelle für Borat- und Phosphationen werden vorzugsweise die entsprechenden Säuren verwendet. Deren Ammonium-Salze oder Salze der einzusetzenden Metalle M können ebenfalls Verwendung finden. Die Quellen für die Metall- oder Ammoniumkationen sowie für die Borat- und Phosphationen löst oder suspendiert man vorzugsweise im durch die Formel (I) gegebenen stöchiometrischen Verhältnis in dem polaren Lösungsmittel. Dies ist so zu verstehen, daß die Einzelkomponenten in solchen Verhältnissen vorliegen müssen, daß Verbindungen der Formel (I) gebildet werden können. Selbstverständlich ist es möglich, einzelne Komponenten im stöchiometrischen Überschuß einzusetzen, so daß nach Reaktionsende der stöchiometrische Überschuß entweder in der Lösung verbleibt oder als Fremdphase die erwünschten Borophosphate der Formel (I) verunreinigt.

Weiterhin betrifft die Erfindung Verfahren zur Herstellung von kristallinen Borophosphaten der allgemeinen Formel (I), dadurch gekennzeichnet, daß man ein Gel herstellt, das die kationischen und die anionischen Komponenten vorzugsweise im durch die Formel (I) angegebenen stöchiometrischen Verhältnis enthält, das Gel vom Lösungsmittel befreit und gewünschtenfalls trocknet und/oder durch Einwirkung von Mikrowellen oder von thermischer Energie sintert.

Die Herstellung kristalliner Festkörper über eine Gelstufe ist auf dem Gebiet der Keramik gut bekannt. Vorzugsweise erfolgt die Herstellung des Gels durch weitere Kondensation eines Sols, was man üblicherweise als Sol-Gel-Prozeß bezeichnet. In der Regel geht man hierbei von hydrolysierbaren Vorstufen aus, die in organischem Medium gelöst sind und durch Zugabe von gegebenenfalls angesäuertem Wasser zu einem Sol hydrolysiert werden. Geeignete Vorstufen hierfür sind Alkyle, Acetylacetonate sowie vorzugsweise Alkoholate der einzusetzenden kationischen Komponenten. Bei geeigneter Kombination der Komponenten kann die Gelbildung jedoch auch aus reiner Wasserphase erfolgen. Dies ist beispielsweise der Fall, wenn man zur Herstellung kristalliner Borophosphate, die als dreiwertige Kationen M Aluminiumionen enthalten, eine gemischte Bor- und Phosphorsäurelösung mit Aluminatlauge neutralisiert. Weiterhin können Gele erhalten werden, wenn man Bor- und Phosphorsäure-haltige Lösungen bzw. Lösungen von Estern dieser Säuren mit Alkalilauge versetzt.

Aus der Chemie der Zeolithe und der Aluminiumphosphate ist es bekannt, daß sich die Poren- bzw. Kanalstruktur der herzustellenden Festkörper bei der Synthese dadurch beeinflussen läßt, daß das Reaktionsmedium sogenannte Template enthält. Damit sind Moleküle gemeint, die durch Vorstrukturierung der Lösung, des Gels und/oder durch Wechselwirkung mit dem wachsenden Kristallgitter dessen dreidimensionale Struktur beeinflussen. Dabei werden diese Templatmoleküle häufig in das Kristallgitter mit eingebaut und können hieraus anschließend durch thermischen Abbau entfernt werden. Die Kristallstruktur der kristallinen Borophosphate der allgemeinen Formel (I) läßt sich ebenfalls durch die Anwesenheit von Templatmolekülen im Reaktionsmedium beeinflussen, ohne daß diese notwendigerweise in die Festkörper eingebaut werden müssen. Nach der Isolierung des Festkörpers lassen sich Templatmoleküle gegebenenfalls dadurch entfernen, daß man die Festkörper nach der Entfernung des Lösungsmittels bei einer Temperatur beispielsweise zwischen 100 und 600°C trocknet bzw. calciniert.

Als Templatmoleküle sind insbesondere primäre, sekundäre oder tertiäre organische Amine oder primäre, sekundäre, tertiäre oder quartäre organische Ammoniumionen oder Phosphoniumionen geeignet. Beispiele hierfür sind Dimethylammoniumionen, Tetramethylammoniumionen, Tetraethylammoniumionen, die als wasserlösliche Salze, vorzugsweise als Chloride, Bromide oder als Hydroxide eingesetzt werden können. Auch Ammoniumionen mit längeren Alkylketten beispielsweise Tetrapropyl- oder Tetrabutylammoniumsalze und höhere Homologe hiervon sind als Templat geeignet.

In einem weiteren Aspekt betrifft die Erfindung die Verwendung von kristallinen Borophosphaten der allgemeinen Formel (I) als Katalysatoren für Reaktionen von Verbindungen mit aziden Wasserstoffatomen und/oder von Estern. Unter Verbindungen mit aziden Wasserstoffatomen sind auch Wasser und anorganische Säuren zu verstehen. Vorzugsweise handelt es sich hierbei jedoch um organische Verbindungen mit aziden Wasserstoffatomen. Beispiele hierfür sind Alkohole, Carbonsäuren und primäre oder sekundäre Amine. Insbesondere sind hierunter Alkohole, Amine und Carbonsäuren fettchemischen Ursprungs zu verstehen. Hierunter sind diejenigen Carbonsäuren zu verstehen, die als Komponenten natürlicher Fette und Öle auftreten, sowie die aus diesen Carbonsäuren ableitbaren Alkohole und Amine. Die Kohlenstoffkettenlängen solcher Carbonsäuren fettchemischen Ursprungs liegen vorzugsweise im Bereich von 8 bis 24 Kohlenstoff-Atomen. Die Kohlenstoff-Ketten, die vorzugsweise linear sind, können dabei gesättigt oder ein- oder mehrfach ungesättigt sein. Darüberhinaus fallen unter Carbonsäuren fettchemischen Ursprungs und daraus abgeleitete Alkohole oder Amine auch solche, die durch Dimerisierung oder Polymerisierung solcher Stoffe erhältlich sind. Beispiele hierfür sind die sogenannten Dimersäuren und die sogenannten Guerbet-Alkohole. Üblicherweise stellen Carbonsäuren, Alkohole und Amine fettchemischen Ursprungs keine Reinsubstanzen dar, sondern weisen je nach Herkunft des pflanzlichen oder tierischen Fettes oder Öls ein charakteristisches Verteilungsmuster der C-Kettenlängen auf.

Als Reaktionskomponenten kommen nicht nur organische Alkohole und/oder Carbonsäuren in Betracht, sondern auch die hieraus durch Umsetzung mit organischen oder anorganischen Säuren bzw. Alkoholen erhältlichen Ester. Bevorzugt werden wiederum solche Ester eingesetzt, bei denen entweder die Säurekomponente oder die Alkoholkomponente fettchemischen Ursprungs ist. Dabei sind wiederum solche Ester bevorzugt, bei denen die Reaktionskomponente entweder anorganisch oder organisch mit einer C-Kettenlänge von 4 oder darunter ist. Beispiele sind Fettsäure-Methylester, Fettsäure-Glyceride und Fettalkoholsulfate.

Die kristallinen Borophosphate der allgemeinen Formel (I) können Reaktionen von Verbindungen mit aziden Wasserstoffatomen und/oder von Estern mit sich selbst oder mit Partnerkomponenten katalysieren, die ebenfalls diesen Stoffgruppen angehören. Bevorzugt werden die Borophosphate jedoch als Katalysatoren für Reaktionen eingesetzt, bei denen die Verbindungen mit aziden Wasserstoffatomen und/oder die Ester mit Oxiranen umgesetzt werden. Das unsubstituierte Oxiran ist in der Technik als Ethylenoxid bekannt. Die einsetzbaren Oxirane können auch alkylsubstituiert sein. Einfachstes Beispiel hierfür ist das Propylenoxid. Die Umsetzung fettchemischer Alkohole, Amine und/oder Carbonsäuren sowie von Estern, die solche Komponenten enthalten, mit Ethylenoxid und/oder Propylenoxid ist ein großtechnisch durchgeführter Prozeß zur Herstellung nichtionischer Tenside. Je nach erwünschtem Anwendungszweck kann dabei das stöchiometrische Verhältnis von Ethylenoxid und/oder Propylenoxid zur fettchemischen Komponente in weiten Grenzen variiert werden, um die Hydrophilie bzw. Hydrophobie der entstehenden oberflächenaktiven Verbindungen gezielt einzustellen.

Weiterhin betrifft die Erfindung die Verwendung von kristallinen Borophosphaten der allgemeinen Formel (I) als Katalysatoren für Reaktionen organischer Verbindungen, bei denen Kohlenstoff-Kohlenstoff-Bindungen gespalten und/oder geknüpft werden. Großtechnisch spielen solche Reaktionen insbesondere bei der Verarbeitung von Erdöl eine Rolle. Die umzusetzenden aliphatischen und/oder aromatischen Kohlenwasserstoffe können jedoch nicht nur als Komponenten von Erdöl, sondern auch als Komponenten von Erdgas und/oder von durch Kohlehydrierung erhältlichen Gemischen vorliegen. Beispiele solcher Reaktionen sind petrochemische Crack- und/oder Reformierungsprozesse, bei denen es sich chemisch vornehmlich um Kettenspaltungen sowie um Alkylierungs- und/oder Acylierungsreaktionen handelt. Als Katalysatoren für solche Reaktionen, die schwerpunktmäßig in Erdölraffinerien durchgeführt werden, werden derzeit vornehmlich unterschiedliche Zeolith-Typen eingesetzt. Durch die erfindungsgemäße Verwendung der kristallinen Borophosphate läßt sich aufgrund der spezifischen Verteilung saurer und basischer Zentren an den äußeren und inneren Festkörperoberflächen die Selektivität solcher Reaktionen beeinflussen. Die Borophosphate unterscheiden sich von den Zeolithen weiterhin durch ihre verringerte Tendenz zur Verkokung und teilweise durch ihre höhere thermische Belastbarkeit, was einerseits höhere Reaktionenstemperaturen ermöglicht, andererseits die Regenerierung der Katalysatoren bei Verkokung erleichtert.

Zur Verwendung als Katalysator können die Produkte erwünschtenfalls nach an sich bekannten Methoden in Formkörper, beispielsweise in Granalien, überführt werden. Die gängigen Methoden hierfür sind dem Verfahrenstechniker bekannt. Sie umfassen beispielsweise die Granulation angefeuchteter Pulver in Extrudern mit anschließender Trocknung, wobei zwischen Extrusion und Trocknung erwünschtenfalls eine Abrundung, beispielsweise auf einem Granulierteller, erfolgen kann. Formkörper lassen sich auch dadurch erhalten, daß man die Produkte trocken oder angefeuchtet in Formen gibt und unter Anwendung von Druck zu Formkörpern verpreßt, wobei höhere Drücke angewendet und damit stabilere Formkörper erhalten werden können als bei dem vorstehend genannten Extrusions-verfahren.

Wünscht man die Porosität der Formkörper zu erhöhen, so kann man bei ihrer Herstellung porenbildende Zusätze zugeben, die nach Ausformung der Formkörper durch Erhitzen entfernt werden. Beispielsweise ist hierfür Holzmehl geeignet, das beim Tempern der Formkörper in einem oxidierenden Gas, beispielsweise in Luft praktisch rückstandslos verbrennt. Weiterhin lassen sich als Porenbildner Ammoniumcarbonat oder Ammoniumhydrogencarbonat einsetzen. Beim Tempern verdampfen diese Salze rückstandsfrei.

In einem weiteren Aspekt betrifft die Erfindung die Verwendung von kristallinen Borophosphaten der allgemeinen Formel (I) als Kationenaustauscher. Wie auch bei den Zeolithen kommen hierfür insbesondere solche Phasen in Betracht, die einwertige Metallionen M oder Ammonium-Ionen enthalten.

In einem weiteren Aspekt betrifft die Erfindung die Verwendung von kristallinen Borophosphaten der allgemeinen Formel (I) als Sorptionsmittel für anorganische oder organische molekulare Verbindungen, insbesondere für polare Moleküle. Dabei kann die Sorption aus einer Gas- oder Flüssigphase erfolgen. Beispiele für solche Sorptionsprozesse sind die Entfernung von Wasser aus organischen Flüssigkeiten oder die Entfernung organischer Verunreinigungen oder von anorganischen gasförmigen Säureanhydriden aus Luft. Bei geeignetem Porendurchmesser können die kristallinen Borophosphate analog den bekannte Zeolithen und Aluminiumphosphaten Molekularsiebeigenschaften haben, d.h. eine besonders hohe Bindungsfähigkeit für Moleküle einer bestimmten Größenordnung aufweisen.

Die Erfindung betrifft ebenso den Einsatz der kristallinen Borphosphate nach Formel (I) als Flammschutzmittel, Rheologieverbesserer oder Füllstoff und Pigment in Papier oder Kunststoffen.

Weiterhin betrifft die Erfindung die Verwendung von kristallinen Borophosphaten der allgemeinen Formel (I) als medizinische Implantate, insbesondere für Zahn- oder Knochenersatz. Diese Verwendung macht sich die hohe Gewebeverträglichkeit der kristallinen Borophosphate zunutze, die u.a. auf die chemische Ähnlichkeit mit Calciumphosphat zurückgeführt werden kann. Besonders geeignet hierfür sind solche Borophosphate der allgemeinen Formel (I), bei denen das Kation M ein Erdalkalimetall-Kation, insbesondere ein Calcium-Ion bedeutet. Dabei können Implantate zum Einsatz kommen, die ganz aus den Borophosphaten bestehen. Es ist jedoch auch möglich und bei Implantaten, die Biegebelastungen ausgesetzt sind, sogar vorzuziehen, die Borophosphate als Beschichtung auf Implantate aus anderen, vorzugsweise metallischen, Materialien aufzubringen und damit beispielsweise das Aufwachsen von Knochensubstanz zu erleichtern. Die Beschichtung kann vollständig oder teilweise erfolgen und bestimmt Oberflächenstrukturen, beispielsweise Vertiefungen, des Trägermaterials erfordern. Beispielsweise kann das Trägermaterial eine offenporige Oberflächenstruktur aufweisen, wobei jeweils der Porengrund mit kristallinem Borphosphat bedeckt ist. Derartige Strukturen mit Hydroxylapatit als Beschichtungsmaterial sind aus der DE-A-42 11 343 bekannt.

### Beispiele

### Synthesen nach dem Sol-Gel-Verfahren

### Beispiel 1

### Kaliumborophosphat

P₄O₁₀ in Ethanol (Molverhältnis: 1:6) wird mit H₃BO₃ und Tetrapropylammoniumbromid (TPA) in Ethanol (Molverhältnis 10:1:50) bei 70°C in einem molaren Verhältnis P:B = 1:1 umgesetzt, auf 90°C erwärmt und 24 h am Rückfluß erhitzt. Nach Abziehen der halben Lösungsmittelmenge werden portionsweise KOH-Plätzchen bis pH=7 zugegeben. Hierbei wird die Lösung gelartig. Nach 4 h am Rückfluß wird das entstandene Produkt mit EtOH gewaschen und bei 150°C getrocknet. Es wird ein feinkristallines weißes Produkt mit einer Korngröße von 2-3 um erhalten. Das Röntgenpulverdiagramm zeigt Reflexe, die keiner bisher bekannten Phase zugeordnet werden können. Nach EDX-Analysen enthält das Produkt B, 0, P und K. Das Produkt ist in Wasser und EtOH schwerlöslich.

### Röntgenpulverdiagramm:

| d(Å) | I/Iₒ |
|---|---|
| 5,562 | 73 |
| 3,734 | 68 |
| 3,517 | 100 |
| 3,356 | 92 |
| 2,910 | 55 |
| 2,760 | 72 |
| 2,170 | 38 |

### Beispiel 2

### Calciumborophosphat

P₄O₁₀ in Ethanol (Molverhältnis 1:6) wird mit H₃BO₃ und Tetrapropylammoniumbromid in Ethanol (Molverhältnis 10:1:50) so vermischt, daß das Molverhältnis P:B = 3:1 ist. Die Lösung wird 3 Stunden am Rückfluß erhitzt. In die auf 70°C abgekühlte Lösung wird so viel Ca(OH)₂-Pulver eingerührt, daß das Molverhältnis Ca:P = 1:1 beträgt. Diese Mischung wird im Rührautoklaven für 10 Stunden bei 150°C unter autogenem Druck gehalten, danach auf Raumtemperatur abgekühlt, filtriert, mit Ethanol gewaschen und bei 110°C getrocknet. Die Elementaranalyse ergibt ein Ca:B:P-Verhältnis von angenähert 3:1:3. Ein Teil des Produkts wurde für 5 Stunden bei 500°C calciniert.

### Beispiel 3

### Na₂[B₇P₃O₁₉]*H₂O

OP(OR)₂OH mit R=n-Butyl und H₃BO₃ werden im molaren Verhältnis 3:1 umgesetzt. Die entstehende Lösung wird mit NaOH-Lsg. auf pH=7 eingestellt und 24 h bei 90°C gehalten. Das entstandene Produkt wird abfiltriert, mit EtOH gewaschen und bei 150°C getrocknet. Das Röntgenpulverdiagramm kann in Anlehnung an die Phase Na₂[B₁₀O₁₆]*4H₂O interpretiert werden. Nach EDX- und AAS-Analysen ist dem Produkt die Summenformel Na₂[B₇P₃O₁₉]*H₂O zuordnen. Das Produkt ist in Wasser leicht, in EtOH jedoch schwerlöslich.

### Röntgenpulverdiagramm:

| d(Å) | I/Iₒ |
|---|---|
| 5,820 | 72 |
| 4,917 | 17 |
| 4,029 | 31 |
| 3,861 | 100 |
| 3,677 | 23 |
| 3,609 | 27 |
| 3,370 | 12 |
| 3,317 | 31 |
| 3,188 | 18 |
| 3,080 | 10 |
| 2,922 | 14 |
| 2,889 | 29 |
| 2,570 | 22 |
| 2,439 | 17 |
| 2,249 | 17 |
| 2,234 | 18 |
| 2,157 | 12 |
| 2,081 | 12 |
| 1,896 | 14 |

### Sorptions- und Desorptionsverhalten

Zur Bestimmung des Sorptions- und Desorptionsverhaltens ausgewählter Prüf-Substanzen wurden diese zunächst im Vakuumtrockenschrank bei 110°C ausgeheizt. In wägegläschen wurden etwa jeweils 10 g der zu prüfenden Substanzen eingewogen und in einer etwa 3 mm hohen Schicht auf dem Boden der wägegläschen verteilt. Zur Prüfung des Sorptionsverhaltens gegenüber Flüssigkeitsdämpfen wurden Schalen mit den zu prüfenden Flüssigkeiten auf den Boden von etwa 2 l fassenden Exsikkatoren gestellt und die wägegläschen mit den Prüfsubstanzen auf die Siebplatte der Exsikkatoren plaziert. Danach wurden die Exsikkatoren geschlossen Um das Sorptionsverhalten gegenüber sauren Gasen zu prüfen, wurden die wägegläschen mit dem Prüfsubstanzen auf die Lochplatten der Exsikkatoren gestellt, über eine Leitung die zu sorbierenden Gase eingeleitet und die Exsikkatoren verschlossen. Nach 24-stündigem Stehen bei Raumtemperatur wurden die Exsikkatoren geöffnet und die Proben zurückgewogen. Die Gewichtszunahme der Proben wurde in Prozent des Probengewichts vor der Sorption ausgedrückt. Um die Desorption zu überprüfen, wurden die beladenen Proben für 2 Stunden bei 110°C im Vakuumtrockenschrank ausgeheizt. Die durch Rückwägen der Proben festgestellte Desorption wurde in Prozent der sorbierten Menge ausgedrückt. Die Ergebnisse sind in Tabelle 1 zusammengestellt.

**Tabelle 1**

| Stoff | Produkt aus Beisp. | Gew.-Zunahme | Desorption bei 110°C |
|---|---|---|---|
| Wasser | 1 | 15 % | 95 % |
| | 2 | 12 % | 98 % |
| Aceton | 1 | 17 % | 100 % |

### Prüfung des Ionenaustauschverhaltens

Zur Überprüfung der Kationenaustauscheigenschaften der Produkte wurde eine Calciumchloridlösung hergestellt, deren Calciumgehalt einer rechnerischen Konzentration von 500 ppm CaCO₃ entsprach. Genau abgewogene Proben von etwa 0,1 g Gewicht wurden zu jeweils 100 ml der Calciumchloridlösung gegeben, bei 25°C 30 Minuten gerührt und danach über ein Membranfilter der Porenweite 0,2 um filtriert. In den Filtraten wurde der Calciumgehalt durch komplexometrische Titration mit EDTA bestimmt und aus der so erhaltenen Resthärte die Calciumbindefähigkeit der Prüfsubstanzen, ausgedrückt in mg CaO pro Gramm Substanz, berechnet.

Ergebnisse: Das Produkt aus dem Beispiel 1 hatte ein Calciumbindevermögen von 35 mg/g.

### Verwendung als Dehydrierungskatalysator für Ringschlußreaktionen

Als Testreaktion wurde die Ringschlußreaktion von Monoethanolamin zu Ethylenimin unter Wasserabspaltung in der Gasphase herangezogen. Als Reaktionsgefäße dienten Rohre aus rostfreiem Stahl mit einem Innendurchmesser von 1,8 cm und einer Länge von 20 cm. Die Rohre wurden mit den auf eine Korngröße zwischen 1 und 2 mm zerkleinerten Prüfsubstanzen gefüllt und in einem Röhrenofen auf 400°C aufgeheizt. Während des Aufheizens wurde mit Stickstoff gespült und dieser Vorgang nach Ende des Aufheizens noch 30 Minuten fortgesetzt. Anschließend wurde dem Stickstoffstrom bei einer Strömungsgeschwindigkeit von etwa 5 l/min 5 Volumenprozent Monoethanolamin zudosiert. Das den Reaktor verlassende Reaktionsgemisch wurde gaschromatographisch analysiert. Die katalytische Ausbeute in Molprozent wurde berechnet durch den Quotienten Mol gebildetes Ethylenimin : Mol eingesetztes Monoethanolamin.

### Katalytische Dehydrierung von Alkoholen

Der vorstehend beschriebene Reaktor wird mit dem auf eine Korngröße zwischen 1 und 2 mm gemahlenen calcinierten Produkt aus Beispiel 2 gefüllt. Unter Spülung mit Stickstoff wird der Reaktor auf 450°C aufgeheizt und nach Erreichen dieser Temperatur für weitere 30 Minuten mit Stickstoff gespült. Anschließend wird bei dieser Temperatur Methanoldampf durch den Reaktor geleitet. Das den Reaktor verlassende Reaktionsgemisch wird durch eine Kühlfalle geleitet, um entstandenes Wasser auszukondensieren. Die gasförmige Fraktion wird gaschromatographisch analysiert. Danach enthält das Reaktionsgemisch neben unreagiertem Methanol als Hauptkomponenten Ethen, Propen und verschiedene Butene. Der Versuch wird mit dem uncalcinierten Produkt aus Beispiel 7 wiederholt, das zuvor auf folgende Weise granuliert wurde: 400 g des Produktes wurden mit 320 g Wasser angefeuchtet und durch einen Extruder gegeben. Der Extruder war so eingestellt, daß Stränge mit einem Durchmesser von 3 mm und etwa 4 mm Länge entstanden. Diese wurden auf einer Pelletiertrommel abgerundet und die Granalien bei 130°C für 3 Stunden getrocknet. Die Dehydrierung von Methanol an diesen Granalien lieferte als wesentliche Produkte ebenfalls Ethen, Propen und verschiedene Butene.

## Patentansprüche

1. Kristalline Borophosphate ein- und/oder mehrwertiger Kationen der allgemeinen Formel (I)
x M₂/ₙO · y B₂O₃ · P₂O₅ · m H₂O (I)
wobei
n 1, 2 oder 3 bedeutet und die Wertigkeit des Metalls M ausdrückt,
y zwischen 0,1 und 50 liegt,
m eine Zahl im Bereich von O bis (2 + y) bedeutet,
x so gewählt wird, daß n·x im Bereich 0,1 bis 6.(1 + y) liegt und
M Natrium oder Kalium bedeutet,
dadurch gekennzeichnet, daß sie ein Röntgenpulverdiagramm mit mindestens den folgenden Linien aufweisen:
| d(Å) | I/Iₒ |
|---|---|
| 5,820 | 72 |
| 4,917 | 17 |
| 4,029 | 31 |
| 3,861 | 100 |
| 3,677 | 23 |
| 3,609 | 27 |
| 3,370 | 12 |
| 3,317 | 31 |
| 3,188 | 18 |
| 3,080 | 10 |
| 2,922 | 14 |
| 2,889 | 29 |
| 2,570 | 22 |
| 2,439 | 17 |
| 2,249 | 17 |
| 2,234 | 18 |
| 2,157 | 12 |
| 2,081 | 12 |
| 1,896 | 14 |
| oder | |
| d(Å) | I/Iₒ |
|---|---|
| 5,562 | 73 |
| 3,734 | 68 |
| 3,517 | 100 |
| 3,356 | 92 |
| 2,910 | 55 |
| 2,760 | 72 |
| 2,170 | 38 |

2. Verfahren zur Herstellung von kristallinen Borophosphaten nach Anspruch 1, dadurch gekennzeichnet, daß die Herstellung des Gels über einen Sol-Gel-Prozeß erfolgt.

3. Verfahren zur Herstellung von kristallinen Borophosphaten der allgemeinen Formel (I) nach Anspruch 1 oder 2, dadurch gekennzeichnet, daß die Herstellung in Gegenwart eines gelösten Templats, vorzugsweise eines sekundären oder tertiären organischen Amins oder von sekundären, tertiären oder quartären organischen Ammoniumionen erfolgt.

4. Verfahren zur Herstellung von kristallinen Borophosphaten der allgemeinen Formel (I) nach einem oder mehreren der Ansprüche 1, 2 und 3, dadurch gekennzeichnet, daß der Festkörper nach Entfernen des Lösungsmittels bei einer Temperatur zwischen 100 und 600°C getrocknet/calciniert wird.

5. Verwendung von kristallinen Borophosphaten der allgemeinen Formel (I) nach Anspruch 1, als Katalysatoren für Reaktionen von Verbindungen mit aziden Wasserstoffatomen und/oder von Estern.

6. Verwendung nach Anspruch 5, dadurch gekennzeichnet, daß es sich bei den Verbindungen mit aziden Wasserstoffatomen um organische, vorzugsweise jeweils fettchemische, Alkohole, Amine und/oder Carbonsäuren handelt.

7. Verwendung nach Anspruch 5, dadurch gekennzeichnet, daß es sich bei den Estern um Ester von Fettsäuren mit Alkoholen oder von Fettalkoholen mit organischen oder anorganischen Säuren handelt.

8. Verwendung nach einem oder mehreren der Ansprüche 5 bis 7, dadurch gekennzeichnet, daß die Verbindungen mit aziden Wasserstoffatomen und/oder die Ester mit, gegebenenfalls substituierten, Oxiranen, vorzugsweise mit Ethylenoxid und/oder Propylenoxid umgesetzt werden.

9. Verwendung von kristallinen Borophosphaten nach Anspruch 1 als Katalysatoren für Reaktionen organischer Verbindungen, bei denen Kohlenstoff-Kohlenstoff-Bindungen gespalten und/oder geknüpft werden.

10. Verwendung nach Anspruch 9, dadurch gekennzeichnet, daß es sich bei den organischen Verbindungen um aliphatische und/oder aromatische Kohlenwasserstoffe, vorzugsweise um Komponenten von Erdöl und/oder von Erdgas und/oder von durch Kohlehydrierung erhältlichen Gemischen handelt.

11. Verwendung nach Anspruch 10 für petrochemische Crack- und/oder Reformierungsprozesse.

12. Verwendung nach einem oder mehreren der Ansprüche 9 bis 11, dadurch gekennzeichnet, daß es sich bei den Reaktionen organischer Verbindungen um Alkylierungs- und/oder Acylierungsreaktionen handelt.

13. Verwendung von kristallinen Borophosphaten nach Anspruch 1 als Kationenaustauscher.

14. Verwendung von kristallinen Borophosphaten nach Anspruch 1 als Sorptionsmittel für, vorzugsweise polare, anorganische oder organische molekulare Verbindungen.

15. Verwendung von kristallinen Borophosphaten als Rheologieverbesserer und zum Flammschutz und als Füllstoffe und Pigmente in Papier oder Kunststoffen.

16. Verwendung von kristallinen Borophosphaten nach Anspruch 1 als medizinische Implantate.

17. Verwendung nach Anspruch 15, dadurch gekennzeichnet, daß es sich bei den medizinischen Implantaten um Zahn- oder Knochenersatz handelt.

18. Verwendung nach einem oder beiden der Ansprüche 15 und 16, dadurch gekennzeichnet, daß die kristallinen Borophosphate der allgemeinen Formel (I) als Beschichtung auf einem anderen, vorzugsweise einem metallischen, Implantatmaterial aufgebracht sind.

## Claims

1. A crystalline borophosphate of monovalent and/or polyvalent cations of the general formula (I)
x M₂/ₙO · y B₂O₃ · P₂O₅ · m H₂O (I)
where
n is 1, 2 or 3 and corresponds to the valence of the metal M,
y is from 0.1 to 50,
m is a number in the range from 0 to (2 + y),
x is selected such that n·x is in the range from 0.1 to 6.(1 + y), and
M is sodium or potassium,
which has an X-ray powder pattern having at least the follow-ing lines:
| d(Å) | I/Iₒ |
|---|---|
| 5.820 | 72 |
| 4.917 | 17 |
| 4.029 | 31 |
| 3.861 | 100 |
| 3.677 | 23 |
| 3.609 | 27 |
| 3.370 | 12 |
| 3.317 | 31 |
| 3.188 | 18 |
| 3.080 | 10 |
| 2.922 | 14 |
| 2.889 | 29 |
| 2.570 | 22 |
| 2.439 | 17 |
| 2.249 | 17 |
| 2.234 | 18 |
| 2.157 | 12 |
| 2.081 | 12 |
| 1.896 | 14 |
| or | |
| d(Å) | I/Iₒ |
|---|---|
| 5.562 | 73 |
| 3.734 | 68 |
| 3.517 | 100 |
| 3.356 | 92 |
| 2.910 | 55 |
| 2.760 | 72 |
| 2.170 | 38 |

2. A process for preparing crystalline borophosphates as claimed in claim 1, wherein the gel is prepared by a sol-gel process.

3. A process for preparing crystalline borophosphates of the general formula (I) as claimed in claim 1 or 2, wherein the preparation is carried out in the presence of a dissolved template, preferably a secondary or tertiary organic amine or secondary, tertiary or quaternary organic ammonium ions.

4. A process for preparing crystalline borophosphates of the general formula (I) as claimed in one or more of claims 1, 2 and 3, wherein the solid is, after removing the solvent, dried/ calcined at from 100 to 600°C.

5. Use of crystalline borophosphates of the general formula (I) as claimed in claim 1 as catalysts for reactions of compounds having acidic hydrogen atoms and/or of esters.

6. Use as claimed in claim 5, wherein the compounds having acidic hydrogen atoms are organic, in each case preferably fatty, alcohols, amines and/or carboxylic acids.

7. Use as claimed in claim 5, wherein the esters are esters of fatty acids with alcohols or of fatty alcohols with organic or inorganic acids.

8. Use as claimed in one or more of claims 5 to 7, wherein the compounds having acidic hydrogen atoms and/or the esters are reacted with unsubstituted or substituted oxiranes, preferably with ethylene oxide and/or propylene oxide.

9. Use of crystalline borophosphates as claimed in claim 1 as catalysts for reactions of organic compounds, in which reactions carbon-carbon bonds are cleaved and/or formed.

10. Use as claimed in claim 9, wherein the organic compounds are aliphatic and/or aromatic hydrocarbons, preferably components of petroleum and/or natural gas and/or mixtures obtainable by hydrogenation of coal.

11. Use as claimed in claim 10 for petrochemical cracking and/or reforming processes.

12. Use as claimed in one or more of claims 9 to 11, wherein the reactions of organic compounds are alkylation and/or acylation reactions.

13. Use of crystalline borophosphates as claimed in claim 1 as cation exchangers.

14. Use of crystalline borophosphates as claimed in claim 1 as sorbents for, preferably polar, inorganic or organic molecules.

15. Use of crystalline borophosphates as rheology improvers, as flame retardants and as fillers and pigments in paper or plastics.

16. Use of crystalline borophosphates as claimed in claim 1 as surgical implants.

17. Use as claimed in claim 16, wherein the surgical implants are tooth or bone replacements.

18. Use as claimed in claim 16 or 17, wherein the crystalline borophosphates of the general formula (I) are applied as a coating to another, preferably metallic implant material.

## Revendications

1. Borophosphates cristallisés à cations mono- et/ou poly-valents, de formule générale I
x M₂/ₙO · y B₂O₃ · P₂O₅ · m H₂O (I)
dans laquelle
n est égal à 1, 2 ou 3 et correspond à la valence du métal M,
y est un nombre allant de 0,1 à 50,
m est un nombre allant de 0 à (2 + y),
x est choisi en sorte que n.x se situe dans l'intervalle de 0,1 à 6. (1 + y) et
M représente le sodium ou le potassium,
caractérisés par le fait qu'ils ont aux rayons X un diagramme de poudre comportant au moins les raies suivantes :
| d(Å) | I/Iₒ |
|---|---|
| 5,820 | 72 |
| 4,917 | 17 |
| 4,029 | 31 |
| 3,861 | 100 |
| 3,677 | 23 |
| 3,609 | 27 |
| 3,370 | 12 |
| 3,317 | 31 |
| 3,188 | 18 |
| 3,080 | 10 |
| 2,922 | 14 |
| 2,889 | 29 |
| 2,570 | 22 |
| 2,439 | 17 |
| 2,249 | 17 |
| 2,234 | 18 |
| 2,157 | 12 |
| 2,081 | 12 |
| 1,896 | 14 |
| ou | |
| d(Å) | I/Iₒ |
|---|---|
| 5,562 | 73 |
| 3,734 | 68 |
| 3,517 | 100 |
| 3,356 | 92 |
| 2,910 | 55 |
| 2,760 | 72 |
| 2,170 | 38 |

2. Procédé de préparation de borophosphates cristallisés selon la revendication 1, caractérisé par le fait que l'on prépare le gel par un procédé du type sol-gel.

3. Procédé de préparation des borophosphates cristallisés de formule générale I selon la revendication 1 ou 2, caractérisé par le fait qu'on les prépare en présence d'une matrice dissoute, de préférence d'une amine organique secondaire ou tertiaire ou d'ions ammonium organiques secondaires, tertiaires ou quaternaires.

4. Procédé de préparation des borophosphates cristallisés de formule générale I selon une ou plusieurs des revendications 1, 2 et 3, caractérisé par le fait que le corps solide est séché/calciné à une température de 100 à 600°C après élimination du solvant.

5. Utilisation des borophosphates cristallisés de formule générale I selon la revendication 1 en tant que catalyseurs pour des réactions entre des composés à atomes d'hydrogène acides et/ou des esters.

6. Utilisation selon la revendication 5, caractérisée par le fait que les composés à atomes d'hydrogène acides sont des alcools, amines et/ou acides carboxyliques organiques, appartenant tous de préférence à la chimie des matières grasses.

7. Utilisation selon la revendication 5, caractérisé par le fait que les esters sont des esters d'acides gras et d'alcools ou d'alcools gras et d'acides organiques ou minéraux.

8. Utilisation selon une ou plusieurs des revendications 5 à 7, caractérisée par le fait que les composés à atomes d'hydrogène acides et/ou les esters sont mis à réagir avec des oxirannes éventuellement substitués, de préférence avec l'oxyde d'éthylène et/ou l'oxyde de propylène.

9. Utilisation des borophosphates cristallisés selon la revendication 1 en tant que catalyseurs pour des réactions de composés organiques dans lesquelles des liaisons carbone-carbone sont scindées et/ou nouées.

10. Utilisation selon la revendication 9, caractérisée par le fait que les composés organiques sont des hydrocarbures aliphatiques et/ou aromatiques, de préférence des composants du pétrole et/ou du gaz naturel et/ou des mélanges obtenus par hydrogénation du charbon.

11. Utilisation selon la revendication 10, pour des opérations pétroléochimiques de craquage et/ou de reformage.

12. Utilisation selon une ou plusieurs des revendications 9 à 11, caractérisée par le fait que les réactions des composés organiques sont des réactions d'alkylation et/ou d'acylation.

13. Utilisation des borophosphates cristallisés selon la revendication i en tant qu'échangeurs de cations.

14. Utilisation des borophosphates cristallisés selon la revendication 1 en tant qu'agents sorbants pour des composés moléculaires minéraux ou organiques de préférence polaires.

15. Utilisation des borophosphates cristallisés en tant qu'additifs améliorant les propriétés rhéologiques et/ou en tant qu'agents ignifugeants et en tant que matières de charge et pigments dans le papier ou les résines synthétiques.

16. Utilisation des borophosphates cristallisés selon la revendication 1, en tant qu'implants médicinaux.

17. Utilisation selon la revendication 15, caractérisée par le fait que les implants médicinaux sont des dents ou des os artificiels.

18. Utilisation selon la revendication 15 ou 16 ou selon les revendications 15 et 16, caractérisée par le fait que les borophosphates cristallisés de formule générale I sont appliqués en revêtement sur une autre matière d'implant, de préférence métallique.
